(19) Europäisches Patentamt
European Patent Office
Office européen des brevets

(11) EP 0 682 024 B1

(12) **EUROPÄISCHE PATENTSCHRIFT**

(45) Veröffentlichungstag und Bekanntmachung des Hinweises auf die Patenterteilung:
**08.12.1999 Patentblatt 1999/49**

(51) Int Cl.[6]: **C07D 471/04**, A61K 31/435
// (C07D471/04, 235:00, 221:00)

(21) Anmeldenummer: **95106760.2**

(22) Anmeldetag: **04.05.1995**

(54) **Cyclohexan-Derivate, Verfahren zu ihrer Herstellung und die Verwendung als Glukose-6-Phosphatase Inhibitoren**

Cyclohexane derivatives, processes for their preparation and their use as glucose-6-phosphatase inhibitors

Dérivés de cyclohexane, procédés pour leur préparation et leur utilisation comme inhibiteurs de glucose-6-phosphatase

(84) Benannte Vertragsstaaten:
**AT BE CH DE DK ES FR GB GR IE IT LI LU NL PT SE**

(30) Priorität: **10.05.1994 DE 4416433**

(43) Veröffentlichungstag der Anmeldung:
**15.11.1995 Patentblatt 1995/46**

(73) Patentinhaber: **HOECHST AKTIENGESELLSCHAFT**
**65929 Frankfurt am Main (DE)**

(72) Erfinder:
- **Hemmerle, Horst, Dr.**
  **D-64653 Lorsch (DE)**
- **Schubert, Gerrit, Dr.**
  **D-65779 Kelkheim (DE)**
- **Burger, Hans-Jörg, Dr.**
  **D-65719 Hofheim (DE)**
- **Herling, Andreas, Dr.**
  **D-65520 Bad Camberg (DE)**
- **Efendic, Suad, Prof. Dr.**
  **S-18134 Lidingö (SE)**

(56) Entgegenhaltungen:
**EP-A- 0 587 087** **EP-A- 0 587 088**
**EP-A- 0 671 392** **EP-A- 0 678 518**

- **PATENT ABSTRACTS OF JAPAN vol. 10 no. 112 (C-342) ,25.April 1986 & JP-A-60 243016 (TSUMURA JIYUNTENDOU K.K.) 3.Dezember 1985,**

Bemerkungen:
Die Akte enthält technische Angaben, die nach dem Eingang der Anmeldung eingereicht wurden und die nicht in dieser Patentschrift enthalten sind.

EP 0 682 024 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

EP-A-0 587 088 betrifft substituierte Cyclohexan-Derivate der Formel A

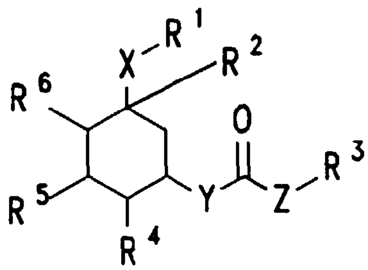

A

die das Glucose-6-Phosphatase-System der Leber in Säugetieren hemmen und daher als Arzneimittel verwendet werden können.

EP-A-O 587 087 beschreibt substituierte Cyclohexanderivate als Hemmer des Glucose-6-Phosphatase-Systems der Leber von Säugetieren.

JP-A-60 243 016 beschreibt substituierte Cyclohexanderivate mit antiviraler Wirkung.

EP-A-0 671 392 beschreibt substituierte Propenderivate als Hemmer des Glucose-6-Phosphatase-Systems der Leber von Säugetieren.

EP-A-0 678 518 beschreibt ein Verfahren zur Herstellung von Cyclohexanderivaten.

In Formel A haben die verschiedenen Reste die folgenden Bedeutungen:

$R^1$: CN, COOH, eine mit einer Schutzgruppe geschützte COOH-Gruppe, $C_1$-$C_4$-Alkanoyl, $SO_3$-$C_1$-$C_4$-Alkyl, $SO_3H$, $SO_2NR^8R^9$, $PO(OH)_2$, PO(OH)(O-$C_1$-$C_4$-Alkyl), $PO(O\text{-}C_1\text{-}C_4\text{-Alkyl})_2$,

$R^2$: $C_1$-$C_{10}$-Alkyl $(R^{11})_n$, O-$C_1$-$C_{10}$-Alkyl $(R^{11})_n$, $C_2$-$C_{10}$-Alkenyl $(R^{11})_n$, O-$C_3$-$C_{10}$-Alkenyl $(R^{11})_n$, $C_2$-$C_{10}$-Alkinyl $(R^{11})_n$, O-$C_3$-$C_{10}$-Alkinyl $(R^{11})_n$, S-$C_1$-$C_{10}$-Alkyl $(R^{11})_n$, S-$C_3$-$C_{10}$-Alkenyl $(R^{11})_n$, S-$C_3$-$C_{10}$-Alkinyl $(R^{11})_n$, NH-$C_1$-$C_{10}$-Alkyl $(R^{11})_n$, NH-$C_3$-$C_{10}$-Alkenyl $(R^{11})_n$ oder NH-$C_3$-$C_{10}$-Alkinyl $(R^{11})_n$, wobei $R^{11}$ gegebenenfalls jeweils mit $R^{12}$ substituiert ist;

$R^3$, $R^{11}$ und $R^{13}$: Alkyl mit 1 bis 10 C-Atomen, Cycloalkyl mit 3-8 Ring-C-Atomen, Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno-, pyridino-, pyrimidino- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $NR^8R^9$, Phenyl, Benzyl, Thienyl, Furyl, Imidazolyl, Pyridyl, O-Phenyl oder O-Benzyl gleich oder verschieden substituiert sein kann, und $R^3$, $R^{11}$ und $R^{13}$ gleich oder verschieden sind;

$R^4$, $R^5$ und $R^6$: H, OH, eine durch übliche Alkoholschutzgruppen geschützte OH-Gruppe, F, Cl, Br oder die für $R^2$ angegebenen Bedeutungen haben, wobei $R^4$, $R^5$ und $R^6$ gleich oder verschieden sind;

$R^7$: $C_1$-$C_4$-Alkyl, Phenyl oder Benzyl;

$R^8$ und $R^9$: H, $C_1$-$C_4$-Alkyl, $C_1$-$C_4$-Alkanoyl, Phenyl, das gegebenenfalls substituiert ist mit F, Cl, Br, J, OH, O-$C_1$-$C_4$-Alkyl, $CF_3$, $-NO_2$ oder CN, wobei $R^8$ und $R^9$ gleich oder verschieden sind, oder $R^8$ und $R^9$ bilden zusammen mit dem Stickstoffatom einen 4 bis 10 gliedrigen, gesättigten heterocyclischen Ring, bei dem gegebenenfalls eine $CH_2$-Gruppe durch O, S oder $NR^{10}$ ersetzt sein kann,

$R^{10}$: H, $C_1$-$C_4$-Alkyl, Phenyl oder Benzyl

$R^{12}$: Phenyl, Naphtyl, Phenanthryl, Pyridyl, Thienyl, Furyl, Pyrimidyl, Indolyl, Imidazolyl, Coumarinyl, Phthaliminyl, Chinolyl, Piperazinyl, Tetrazolyl, Triazolyl, Oxazolyl oder deren thieno- oder benzoanellierte Derivate, wobei der Aromat bzw. Heteroaromat ein- oder mehrfach mit F, Cl, Br, J, OH, $CF_3$, $-NO_2$, CN, $C_1$-$C_4$-Alkoxy, $C_1$-$C_4$-Alkyl, $C_2$-$C_4$-Alkenyl, $NR^8R^9$, Phenyl, Benzyl, Thienyl, Furyl, Imidazolyl, Pyridyl, O-Phenyl oder O-Benzyl gleich oder verschieden substituiert sein kann;

X: $(CH_2)_m$, -CH = CH-, -C≡C-, $-CH_2-O-CH_2-$, $-CH_2-S-CH_2-$ oder

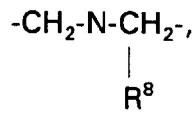

Y: $(CH_2)_m$, O, S, $NR^8$,

Z: $(CH_2)_m$, S, O, S-$C_1$-$C_{10}$-Alkyl, O-$C_1$-$C_{10}$-Alkyl, CH=CH, CH=CF, CH =CCl, CH=CBr, $CH_2$-CO, $CH_2$-CHF, $CH_2$-CHCl, $CH_2$-CHBr,

$CH_2$-CHJ, $C_3$-$C_{10}$-Cycloalkylen, $C_3$-$C_{10}$-Cycloalkenylen, wobei 1 bis 3 Ring-C-Atome durch Schwefel-, Sauerstoff- oder Stickstoffatome ersetzt sein können, $COOR^7$, C≡C, CH =C($C_1$-$C_4$-Alkyl), CH = C(CN), CH =C($NR^8R^9$), CH =C($C_1$-$C_4$-Alkanoyl), CH = C($R^{13}$), $NR^8$ und wenn Y Sauerstoff ist, kann

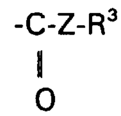

gemeinsam einen Aminosäurerest, ausgewählt aus der Gruppe bestehend aus Ala, Arg, Asn, Asp, Cys, Gln, Glu, Gly, His, Ile,Leu, Lys, Phe, Pro, Ser, Thr, Trp, Tyr und deren durch übliche Schutzgruppen geschützte Derivate, darstellen,

n: null, 1 oder 2

m: null, 1, 2, 3 oder 4.

Es wurde nun völlig überraschend gefunden, daß Verbindungen der Formel A, welche ganz spezielle Reste tragen und in EP-A-0 587 088 nicht beschrieben sind, eine außerordentlich starke Hemmwirkung auf das Glucose-6-Phosphatase-System der Leber in Säugern aufweisen.

Die Erfindung betrifft daher Cyclohexan-Derivate der Formel

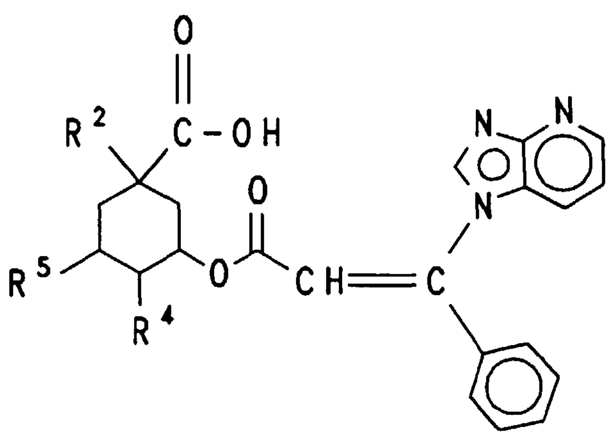

I

worin

$R^2$ O-$C_3$-$C_5$-Alkyl ($R^{11}$) ist, wobei der Alkylteil geradkettig, verzweigt oder cyclisch ist und

$R^{11}$ Phenyl, das gegebenenfalls in 4-Stellung mit Fluor, Chlor oder Methyl substituiert ist, und

$R^4$ und $R^5$ gleich oder verschieden sind und H oder OH bedeuten, sowie deren physiologisch verträgliche Salze.

Die erfindungsgemäßen Verbindungen der Formel I enthalten eine Reihe von Stereozentren. Die Erfindung betrifft alle möglichen Enantio- und Diastereomere. Sie werden alle durch die Formel I wiedergegeben.

Die Wirkung der erfindungsgemäßen Verbindungen auf das Glucose-6-Phosphatase-Sytem wurde in einem Enzymtest in Lebermikrosomen untersucht.

Für die Präparation der die Glucose-6-Phosphatase enthaltenden Mikrosomen-Fraktion wurden frische Leber-Organe von männlichen Wistar-Ratten verwendet und wie in der Literatur beschrieben aufgearbeitet [Canfield, W. K. und Arion, W. J., J. Biol. Chem. 263, 7458-7460, (1988)]. Diese Mikrosomen-Fraktion kann bei -70 °C mindestens 2 Monate lang ohne signifikanten Aktivitätsverlust aufbewahrt werden.

Der Nachweis der Glucose-6-Phosphatase-Aktivität wurde wie in der Literatur angegeben (Arion, W. J. in Methods Enzymol. 174, Academic Press 1989, Seite 58-67) durch Bestimmung des aus Glucose-6-Phosphat freigesetzten Phosphats durchgeführt. 0,1 ml Testansatz enthielten Glucose-6-Phosphat (1 mMol/l), die Testsubstanz, 0,1 mg Mikrosomen-Fraktion und 100 mMol/l HEPES-Puffer (4-(2-Hydroxyethyl)piperazin-1-ethansulfonsäure), pH 7,0. Die Reaktion wurde durch Zugabe des Enzyms gestartet. Nach Ablauf von 20 min bei Raumtemperatur wurde die Reaktion durch Zugabe von 0,2 ml Phosphat-Reagens gestoppt. Die Probe wurde 30 min lang bei 37 °C inkubiert, und die Absorption (A) der blauen Farbe anschließend bei 570 nm gemessen. Die inhibitorische Wirksamkeit der Testsubstanz ergab sich durch Vergleich mit einer Kontroll-Reaktion, die keine Testsubstanz enthielt nach der Formel

$$\text{Prozent Hemmung} = \frac{\text{A(Kontrolle) - A(Testsubstanz)}}{\text{A(Kontrolle)}} \text{ X } 100$$

Sofern erforderlich wurde die hemmende Wirkung der Testsubstanz als Funktion der eingesetzten Konzentration der Testsubstanz ermittelt, und daraus die Konzentration für die 50 %ige Hemmung der Enzymaktivität ($IC_{50}$) berechnet.

Für die nachfolgend aufgeführten Verbindungen wurde der $IC_{50}$-Wert ermittelt:

Beispiel 1

$IC_{50}$

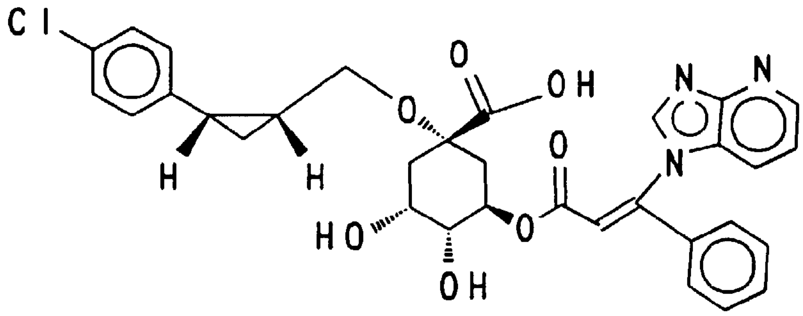

$2 \cdot 10^{-9}$ M

Beispiel 2

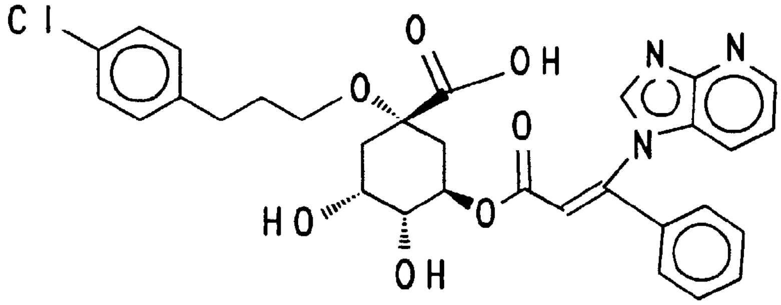

$13 \cdot 10^{-8}$ M

Beispiel 3

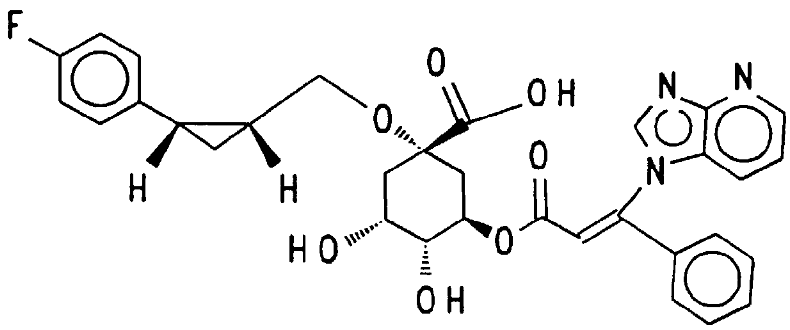

$4 \cdot 10^{-9}$ M

Beispiel 4

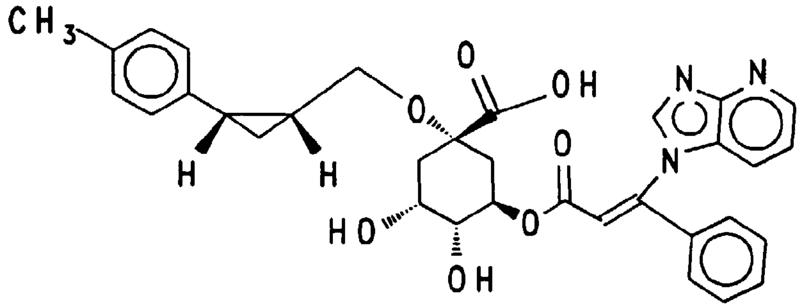

$6 \cdot 10^{-9} M$

Beispiel 5

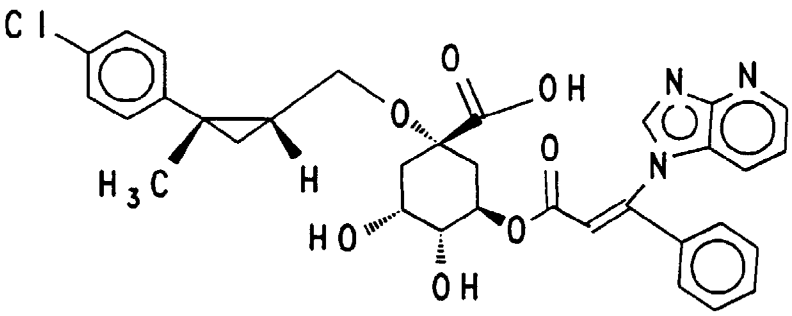

$3 \cdot 10^{-9} M$

Beispiel 6

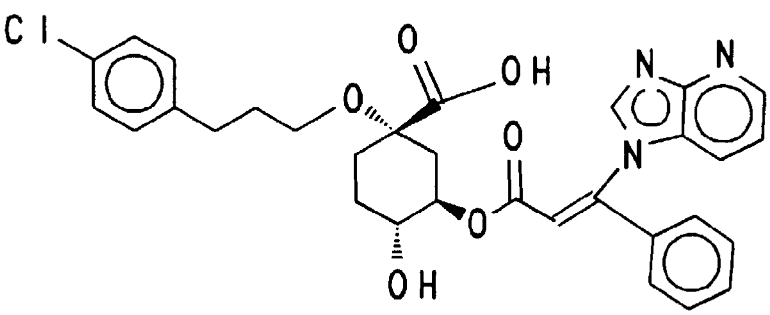

$1.0 \cdot 10^{-8} M$

Beispiel 7

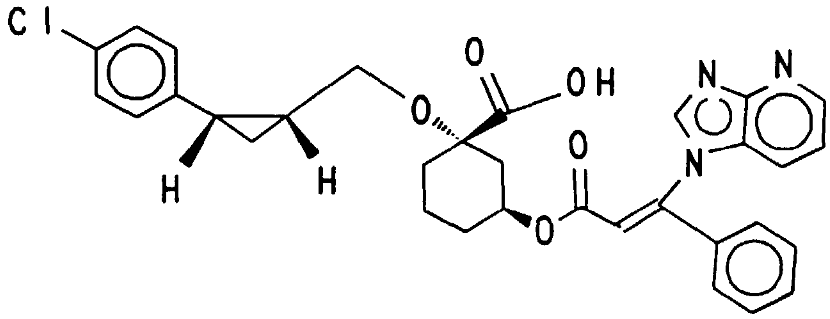

$4 \cdot 10^{-8}$ M

Die erfindungsgemäßen Verbindungen stellen also eine Auswahl aus den in EP-A-0 587 088 beanspruchten Verbindungen dar.

Die Herstellung der erfindungsgemäßen Verbindungen der Formel I erfolgt analog dem in EP-A-0 587 088 beschriebenen Verfahren, wobei im Falle $R^2$ = O-$C_3$-$C_5$-Cycloalkyl ($R^{11}$) vorteilhaft analog dem in EP-A-0 678 518 vorgeschlagenen Verfahren zur Herstellung des Ausgangsproduktes gearbeitet wird.

Beispiel 1

1. Herstellung der Ausgangsverbindung 1e

Stufe a:

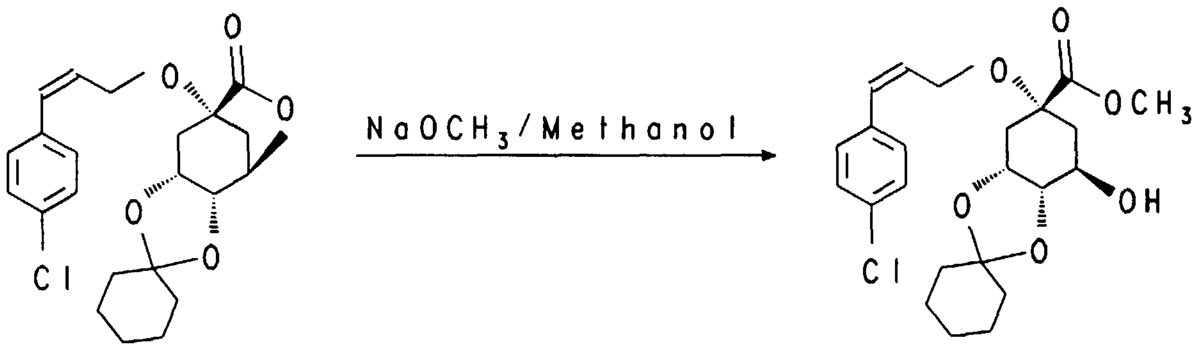


(1a) (1b)

Zu einer Lösung von 5 l wasserfreiem Methanol und 1.5 l wasserfreiem Tetrahydrofuran wurde unter Argon-Atmosphäre bei Raumtemperatur 2.95 g (0.0985 Mol) NaH, 80%ig, portionsweise zugegeben. Anschließend gab man ebenfalls bei Raumtemperatur Verbindung 1 (vergl. EP-A-0 587 088) als Feststoff zu. Nach 3-4 Stunden erhielt man eine klare Lösung. Zur Aufarbeitung gab man 6.0 g Eisessig (pH~5) und danach portionsweise 2 l Wasser zu. Es bildete sich ein flockiger Niederschlag von nicht umgesetztem Lacton, der sich problemlos abfiltrieren ließ. (Rückgewinnung von Edukt!)
Anschließend engte man das Filtrat solange ein, bis sich ein dicker weißer Niederschlag bildete. Man kühlte mit Eis ab, saugte den Niederschlag ab und wusch mit eiskaltem Methanol/Wasser 1:1 nach.
Nach Trocknen des Niederschlags bei 1 mbar und 40°C erhielt man 370 g (86%) Verbindung 1b als farblosen Feststoff. Fp.: 102-104°C

Stufe b:

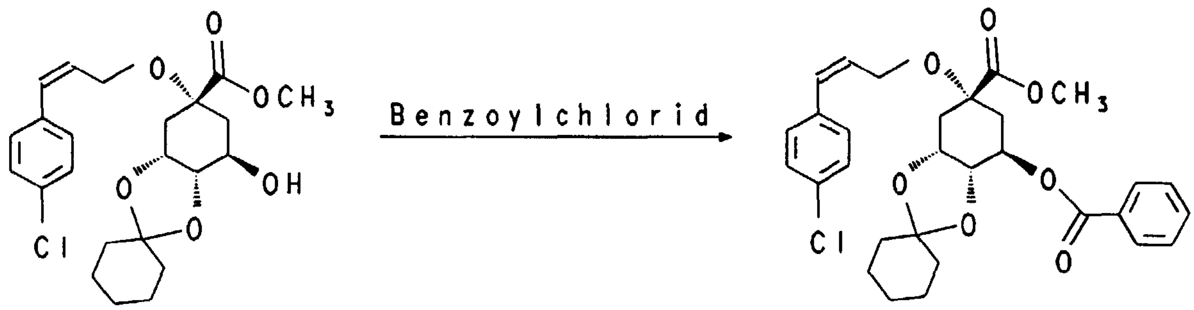


(1b) (1c)

384.0 g (0.879 Mol) Alkohol 1b aus Stufe a wurden in 1.3 l wasserfreiem Dichlormethan gelöst. Anschließend wurden 10.74 g (0.0879 Mol) 4-Dimethylaminopyridin sowie 364.8 ml (2.637 Mol) wasserfreies Triethylamin zugegeben. Man kühlte die Lösung auf 0-10°C ab und tropfte 164.7 ml (1.418 Mol) Benzoylchlorid gelöst in 350 ml wasserfreiem Dichlormethan zu. Nach 4 Stunden bei Raumtemperatur waren nur noch Spuren Edukt vorhanden.
DC: Essigester/Cyclohexan 1:2
Man gab das Reaktionsprodukt auf 1.5 l Wasser / 400 g $NH_4Cl$ / 1 l Eis. Anschließend extrahierte man zweimal mit Dichlormethan, wusch einmal mit gesättigter Bicarbonat-Lösung und trocknete die vereinigten organischen Phasen mit $Na_2SO_4$. Nach dem Einengen wurde der Rückstand aus Isopropanol kristallisiert.
Man erhielt 437.0 g (91.9 %) Produkt 1c.
Fp.: 104-107°C

Stufe c:

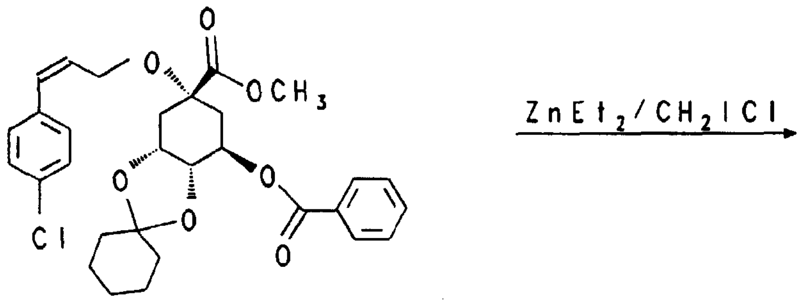


(1c)

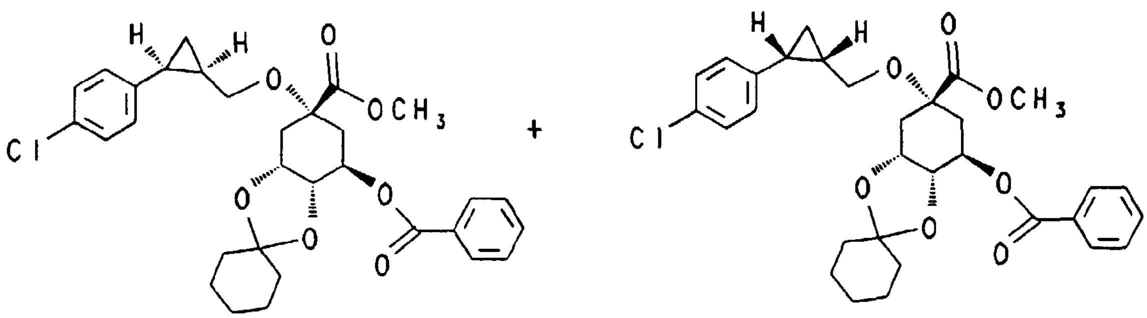

(1d) (1d')

| | | |
|---|---|---|
| Nach Kristallisation | 5 | 1 |
| | >98 | <2 |

Zu 2 l wasserfreiem Dichlorethan ** wurden bei 0°C unter Argon-Atmosphäre 80.5 ml (0.785 Mol) reines Diethylzink * aus Stahlbombe mit Hilfe einer Stahlkanüle übergedrückt.
Anschließend tropfte man bei 0-10°C 114.4 ml (1.57 Mol) Chloriodmethan zu, rührte die entstandene Suspension 30 Minuten nach und tropfte danach 169.89 g (0.314 Mol) des Olefins 1c aus Stufe b, gelöst in 500 ml wasserfreiem Dichlorethan, zu. Nach 1 Stunde bei 0-10°C ließ man das Reaktionsgemisch auf Raumtemperatur aufwärmen und rührte weitere 3 Stunden bei Raumtemperatur.
Das Reaktionsgemisch wurde unter Stickstoff-Atmosphäre langsam in eine Lösung von 300 g $NH_4Cl$ / 1.5 l Eiswasser gegossen und mit Dichlormethan extrahiert. Die vereinigten organischen Phasen wurden mit gesättigter NaCl-Lösung ausgeschüttelt und mit $Na_2SO_4$ getrocknet.
Anschließend engte man im Vakuum den größten Teil des Lösungsmittels ab und verdünnte mit Isopropanol. Danach engte man weiter ein, bis ein dicker Niederschlag ausfiel. Man saugte diesen Niederschlag ab und kristallisierte zweimal aus Isopropanol um und erhielt 109.8 g (63%) Verbindung 1d mit einem DE 98 %.
Fp.: 143-144°C

** statt Dichlorethan wurden auch andere inerte Lösungsmittel verwendet (Dichlormethan, Toluol, THF).
* statt reinem Diethylzink kann auch 1 molare Lösung in Toluol (Aldrich) verwendet werden.

Stufe d:

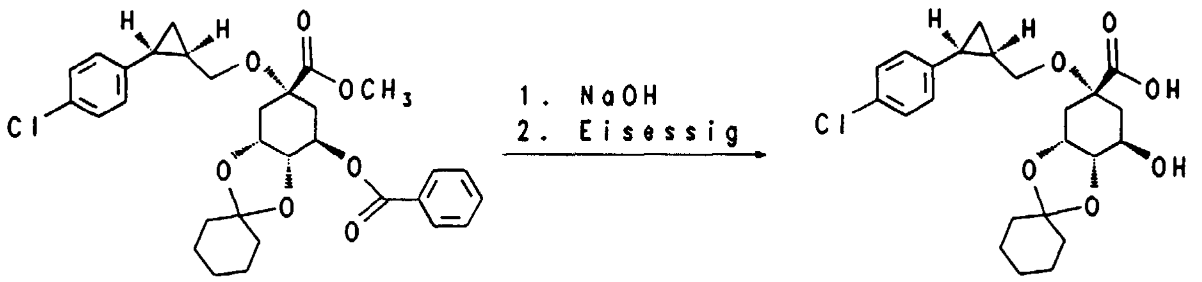


(1d)

(1e)

99.0 g (0.178 Mol) Verbindung 1d aus Stufe c wurden in 1200 ml Dioxan gelöst und 890 ml 2 N Natronlauge wurden zugesetzt. Die Suspension wurde 2 Stunden auf 80°C erwärmt.
Die Reaktionslösung wurde auf ca. 10°C heruntergekühlt und 228 ml (2 Mol) halbkonz. Eisessig langsam zugetropft (pH 5-6). Anschließend wurde die Lösung soweit einrotiert bis die erste Trübung auftrat. Dieses Konzentrat wurde unter kräftigem Rühren auf ca. 1500 ml Wasser gegossen, aus dem nach 10 Minuten Rühren ein kristalliner Niederschlag ausfiel. Dieser Niederschlag wurde abgesaugt und im Vakuum bei 22°C und 0.5 bar getrocknet. Man erhielt 82,2 g Verbindung 1e.

2. Herstellung der Ausgangsverbindung der Formel 1F

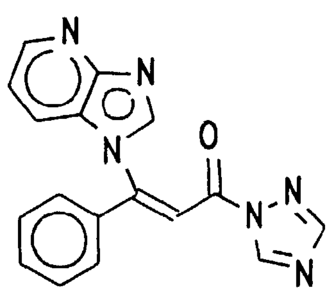

1 F

erfolgt analog den Angaben in EP-A-0 587 088 gemäß folgendem Reaktionsschema:

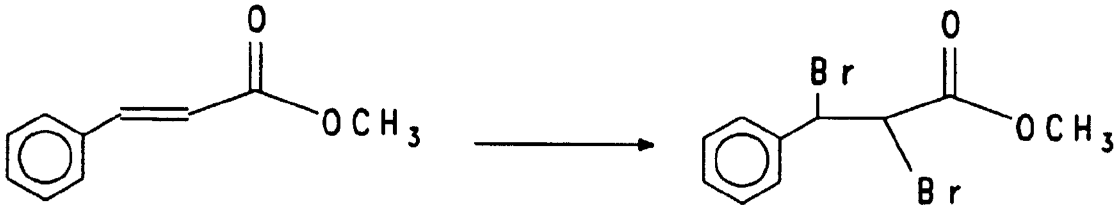

1 A 1 B

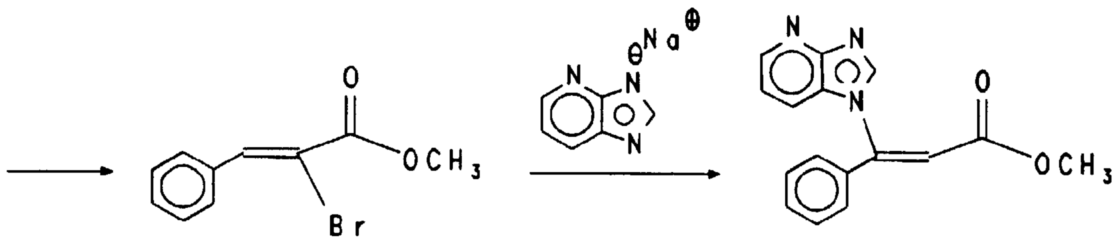

1 C 1 D

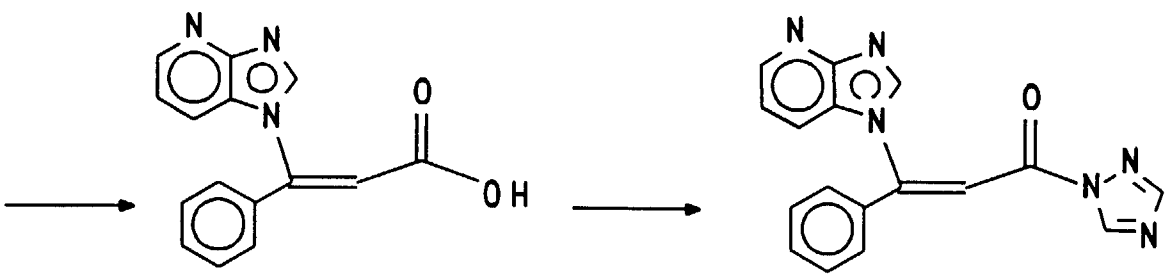

1 E 1 F

Darstellung von 1B aus 1A:

162 g (1 Mol) 1A werden in 400 ml Dichlormethan gelöst. 31,4 ml Brom werden bei 0 - 15°C zugetropft. Man rührt 2 h bei 15°C und engt im Vakuum ein. Man erhält 261 g 1B als farblosen Feststoff.

Darstellung von 1D aus 1B:

Ein Gemisch von 50 g 2,3-Dibrom-3-phenylpropansäuremethylester 1B, 100 ml Triethylamin und 500 ml Toluol wird 1 h zum Sieden erhitzt, anschließend auf Raumtemperatur gekühlt und filtriert. Das Filtrat wird im Vakuum eingedampft und die so erhaltene α-Bromzimtsäure 1C ohne Reinigung weiterverwendet. Zu einer Suspension von 4,7 g NaH (80 %ig in Mineralöl) in 100 ml wasserfreiem DMF werden 0,2 mol Imidazopyridin, gelöst in 150 ml wasserfreiem DMF, unter Rühren zugetropft. Die Temperatur des Gemisches wird dabei durch Kühlung mit Eis unter 35°C gehalten. Nach beendeter Zugabe wird 1 h bei Raumtemperatur gerührt. Die zuvor hergestellte α-Bromzimtsäure wird in 200 ml wasserfreiem DMF gelöst und unter Kühlung mit Eis die Lösung des Azol-Natriumsalzes unter Rühren zugetropft. Nach zweistündigem Rühren bei Raumtemperatur werden 10,8 ml Eisessig zugegeben, das Gemisch wird in 1,5 l Eiswasser eingerührt, mehrfach mit Ethylacetat extrahiert und die organischen Phasen werden mit Wasser gewaschen. Die organischen Phasen werden getrocknet, im Vakuum eingedampft und der Rückstand aus Essigester kristallisiert. Man erhält 37,0 g 1D als hellgelbe Kristalle.

Darstellung von 1E aus 1D:

27,0 g (0,097 Mol) 1D wurden in 200 ml Methanol gelöst. 100 ml 2,5 N NaOH wurden zugegeben. Man rührt diese Lösung 12 h bei 22°C. Anschließend tropft man 200 ml 2 N Essigsäure zu. Der ausgefallene Niederschlag wurde abgesaugt und mit Essigester gewaschen. Man erhält 22,0 g 1E als farblosen Feststoff.

Darstellung von 1F aus 1E:

9,2 g (34,7 mmol) 1E wurden in 170 ml Dimethylformamid gelöst und bei 22°C 6,5 g (38,2 mmol) Carbonylditriazol zugesetzt. Man erhitzte die Suspension 1 Stunde auf 50-60°C und erhielt eine klare gelbe Lösung von 1F in Dimethylformamid, die ohne Aufarbeitung in der Folgereaktion eingesetzt wurde.

3. Herstellung von

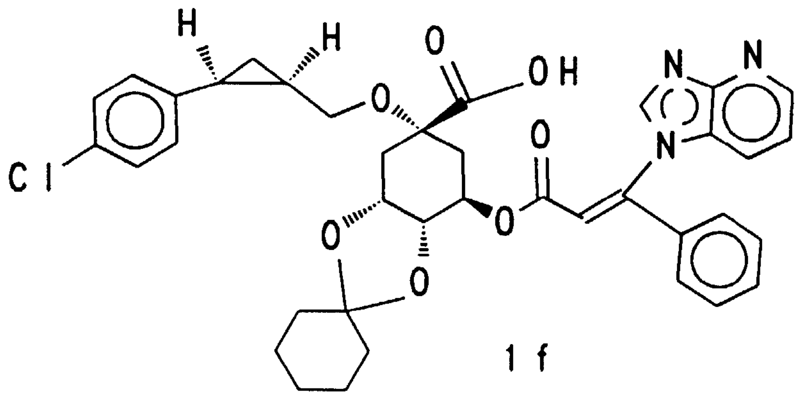

1 f

aus 1e und 1F

10,1 g (23,1 mmol) le werden in 200 ml wasserfreiem Dimethylformamid gelöst und bei 0 - 5°C 2,08 g 80 %iges Natriumhydrid zugegeben. Es bildet sich eine Suspension des Natriumsalzes zu dem 1,5 eq. der Lösung 1F bei 0 - 5°C getropft werden. Nach 1 h wird die Reaktionslösung auf 2 N Essigsäure gegeben. Man extrahiert mit Essigester und trocknet die vereinigten organischen Phasen mit Natriumsulfat. Nach dem Einengen im Vakuum wird der Rückstand durch Chromatographie an Kieselgel (Laufmittel Essigester/n-Heptan/Methanol/Eisessig 8:10:1:1) gereinigt. Man erhält 5,6 g (8,19 mol) 1f als farbloses zähes Öl.

4. Herstellung der Verbindung 1

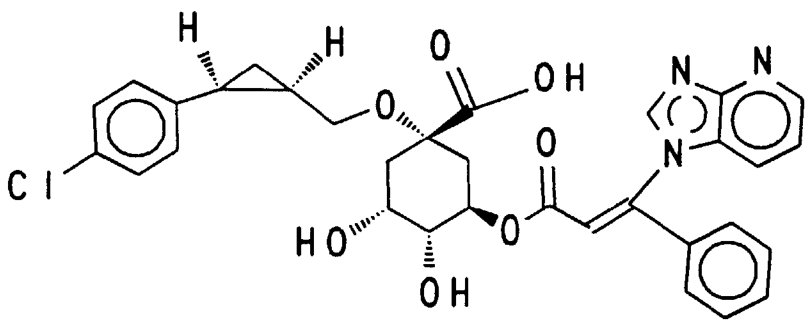

1

Darstellung von 1 aus 1f:

21,0 g 1f werden in 300 ml Dioxan gelöst und 75 ml 2 N HCl zugegeben. Man erwärmt diese Lösung 2 1/2 Stunden auf 50 - 60°C und kühlt danach wieder auf 20°C ab. Anschließend tropft man 135 ml 1 N Natronlauge zu (pH 3). Man destilliert das Dioxan im Vakuum ab und erhält eine wäßrige Suspension. Man saugt den Niederschlag ab. Man erhält 13,8 g 1 als farblosen Feststoff. F.p. 158-160°C

In analoger Weise wurden die folgenden Verbindungen hergestellt.

Beispiel 2

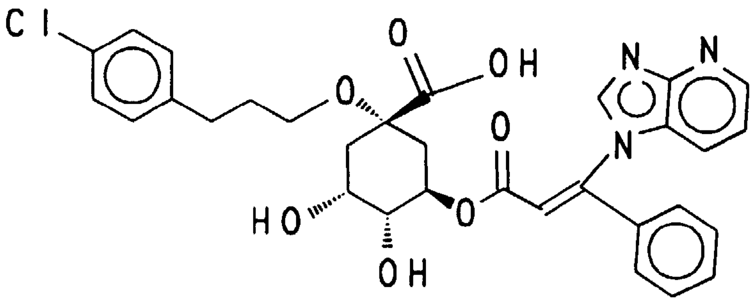

MS: m/z = 592 (M + H$^+$)

Beispiel 3

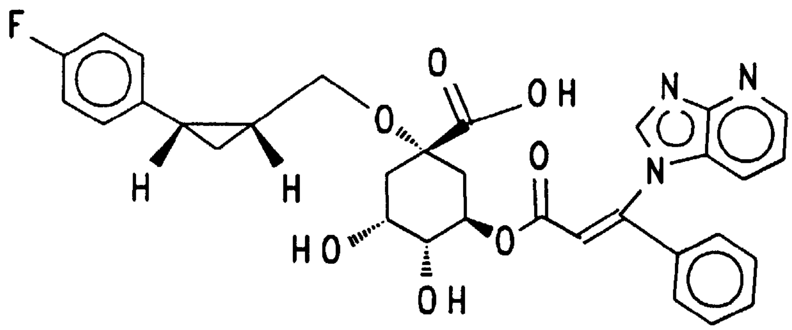

Fp.: 132 - 135°C

Beispiel 4

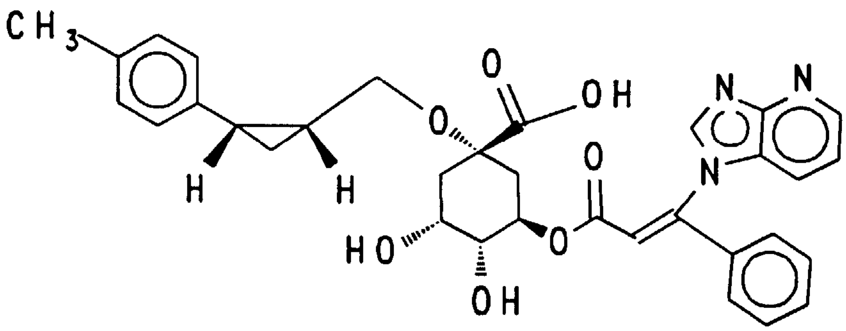

MS: m/z = 583 (M + H+)

Beispiel 5

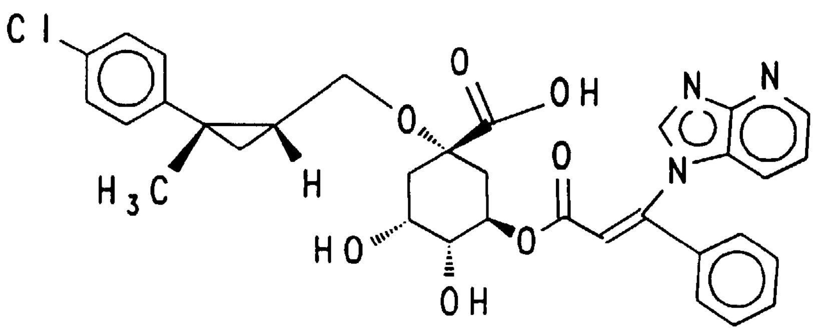

MS: m/z = 618 (M + H+)

Beispiel 6

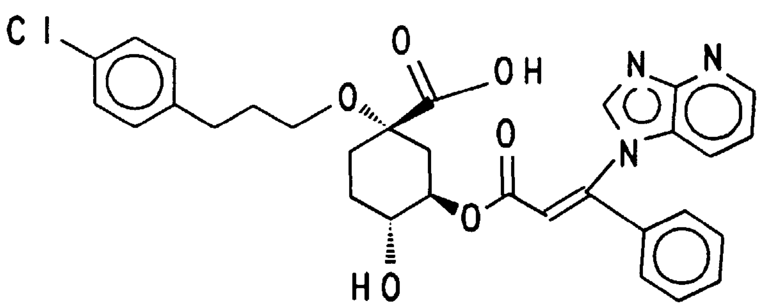

m/z = 576 (M + H+)

Beispiel 7

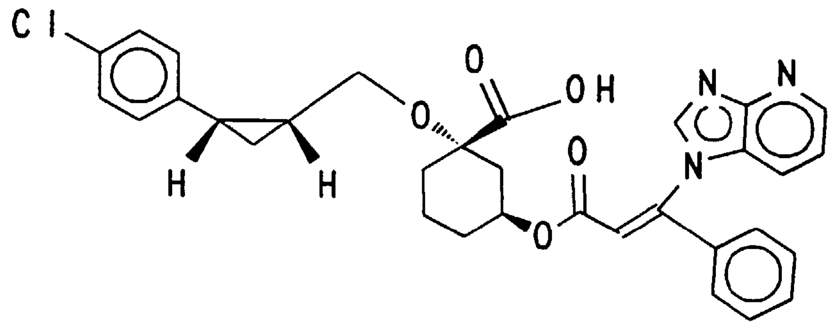

m/z = 572 (M + $H^+$)

## Patentansprüche

1. Cyclohexan-Derivate der Formel I

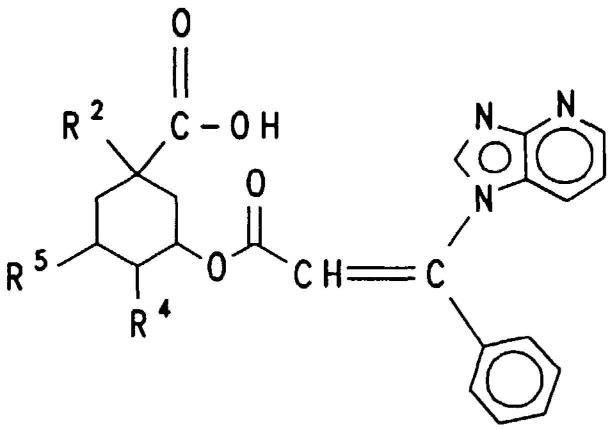

I

worin

| | |
|---|---|
| $R^2$ | O-$C_3$-$C_5$-Alkyl ($R^{11}$) ist, wobei der Alkylteil geradkettig, verzweigt oder cyclisch ist und |
| $R^{11}$ | Phenyl, das gegebenenfalls in 4-Stellung mit Fluor, Chlor oder Methyl substituiert ist, und |
| $R^4$ und $R^5$ | gleich oder verschieden sind und H oder OH bedeuten, |

sowie deren physiologisch verträgliche Salze.

2. Anwendung von Verbindungen nach Anspruch 1 zur Herstellung eines Medikaments zur Behandlung von Krankheiten, die mit einer erhöhten Aktivität des Glucose-6-Phosphatase-Systems verbunden sind.

3. Anwendung von Verbindungen nach Anspruch 1 zur Herstellung eines Medikaments zur Behandlung von Krankheiten, die mit einer erhöhten Glucoseproduktion der Leber verbunden sind.

4. Anwendung von Verbindungen nach Anspruch 1 zur Herstellung eines Medikaments zur Behandlung des Typ II Diabetes (nicht insulinabhängiger oder Altersdiabetes).

5. Arzneimittel enthaltend eine Verbindung nach Anspruch 1.

## Claims

1. A cyclohexane derivative of the formula I

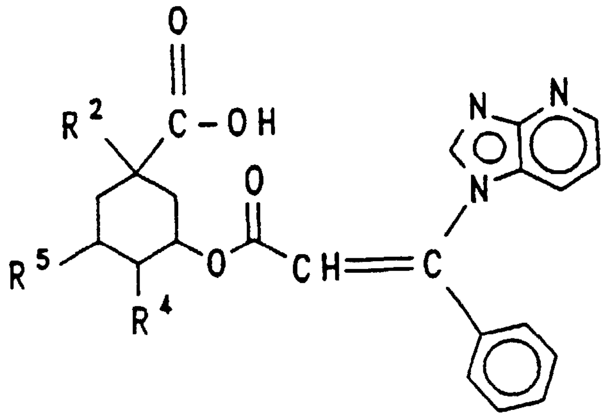

I

wherein

| | |
|---|---|
| $R^2$ | is O-$C_3$-$C_5$-alkyl ($R^{11}$), where the alkyl moiety is straight-chain, branched or cyclic and |
| $R^{11}$ | is phenyl which is optionally substituted in the 4-position by fluorine, chlorine or methyl, and |
| $R^4$ and $R^5$ | are identical or different and are H or OH, |

and its physiologically tolerable salts.

2. The use of compounds as claimed in claim 1 for the production of a medicament for the treatment of diseases which are associated with an increased activity of the glucose-6-phosphatase system.

3. The use of compounds as claimed in claim 1 for the production of a medicament for the treatment of diseases which are associated with an increased glucose production of the liver.

4. The use of compounds as claimed in claim 1 for the production of a medicament for the treatment of type II diabetes (non-insulin-dependent or adult-onset diabetes).

5. A pharmaceutical containing a compound as claimed in claim 1.

## Revendications

1. Dérivés de cyclohexane de la formule I :

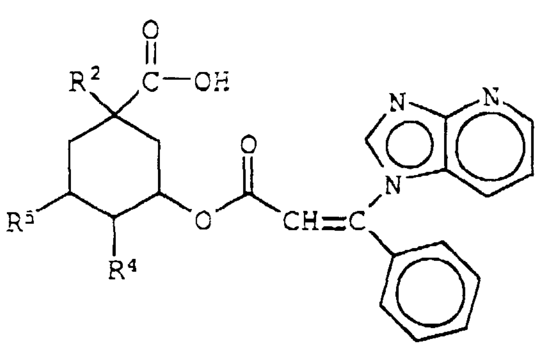

I

dans laquelle :

$R^2$ est O-alkyle en $C_3$-$C_5$ ($R^{11}$), la partie alkyle étant à chaîne droite ou ramifiée, ou cyclique, et
$R^{11}$ est un phényle, le cas échéant substitué en position 4 par un fluor, chlore ou méthyle, et
$R^4$ et $R^5$ sont identiques ou différents et signifient H ou OH,

ainsi que leurs sels physiologiquement compatibles.

**2.** Utilisation de composés selon la revendication 1 pour la préparation d'un médicament pour le traitement de maladies associées à une activité élevée du système glucose-6-phosphatase.

**3.** Utilisation de composés selon la revendication 1 pour la préparation d'un médicament pour le traitement de maladies associées à une production élevée de glucose par le foie.

**4.** Utilisation de composés selon la revendication 1 pour le traitement du diabète de type II (diabète non insulinodépendant ou diabète de vieillesse).

**5.** Médicaments contenant un composé selon la revendication 1.